# EUROPEAN PATENT APPLICATION

(11) **EP 1 785 723 A1**
(43) Date of publication of application: **16.05.2007**
(21) Application number: 05256977.9
(22) Date of filing: 11.11.2005
(51) Int. Cl.: G01N 25/20, G01N 33/48, G01K 17/00, C12Q 1/04, A61B 5/00

(54) **Micro calorimetric analysis of mammalian cell susceptibility to agents that affect cell growth, metabolism and immune response, and use of micro calorimetric analysis in a transplantation test**

(71) Applicant: Stratos Bio Ltd., 1205 Geneva (CH)
(72) Inventor: Kjeldsen, Naja Joslin, 4055 Basel (CH)
(74) Representative: Benson, John Everett

(57) **Abstract**

Method of determining whether or not an organ for transplantation (from a human donor) or xenotransplantation (from an animal donor) is infected with a microoorganism (which is optionally a virus) comprising
- measuring heat output from the organ to detect activity of said microorganism, or
- forcing expression of latent DNA by using agents that stimulate m-RNA synthesis,

to thereby determine the presence of a microorganism in the organ.

## Description

### Field of the Invention

The invention relates to microcalorimetric analysis of agents used for either stimulating or repressing the immune system, such as anti-retroviral agents and agents used for transplantation. The present invention also relates to use of microcalorimetry to test for cell necrosis and rejection of organs subsequent to transplantation and elucidate the mode of action of already existing anti rejection agents such as cyclosporins both alone and in combinations. Further the invention relates to
- use of microcalorimetry to develop new agents either alone or in combination to prevent rejection
- use of microcalorimetry to assess organs for viral vectors prior to transplantation by stimulating eukaryotic mRNA synthesis and expression of latent DNA
- use of microcalorimetry to elucidate the mode of action of anti retroviral agents and their protective effect on DNA expression and stimulation of RNA synthesis.

### Background of the Invention

There is a need for improved methods of testing organs for transplantation and to further investigate agents used to treat transplantation patients.

### Summary of the Invention

Microcalorimetric studies of both growing and respiring bacteria and antibiotic action of those have concluded that agents that interfere with RNA synthesis may be responsible for both cell growth and expression of latent DNA, which is an important factor when attempting xenotransplantation.

These findings can also be applied to transplantation in that immuno suppressive agents such as cyclosporin can be tested in living cell systems both alone and in combinations and can give early warning on cell necrosis and potential rejection episodes as well as immunosuppressant effect.

They can also be applied for assessment of agents that stimulate the immune system such as anti-retroviral agents used for treatment of HIV positive patients

The invention concerns, amongst other aspects, use of micro/nano calorimetry to detect viruses in an organ for transplantation. Accordingly the invention provides a method of determining whether or not an organ for transplantation (from a human donor) or xenotransplantation (from an animal donor) is infected with a microoorganism (which is optionally a virus) comprising
- measuring heat output from the organ to detect activity of said microorganism, and/or
- forcing expression of latent DNA by using agents that stimulate mRNA synthesis,
to thereby determine the presence of a microorganism in the organ.

### Detailed description of the invention

Herein the term "microorganism" is understood to include viruses unless the context requires otherwise.

The invention provides a method of determining whether or not an organ for transplantation comprises (for example is infected with) a microorganism (for example a virus) comprising measuring heat output from the organ to detect presence of the microorganism and to thereby determine the presence of a virus in the organ. The heat output is typically due to an activity of the microorganism.

The organ may be from a human donor or from an animal donor. The organ may be from a human donor for transplantation into a human donor. The organ may be from an animal donor for transplantation into a human donor (i.e. for xenotransplantation). Such an animal is typically a mammal, such as a primate or pig.

The invention also provides a method of determining the suitability of a donor organ for transplantation to a patient comprising adding in vitro a portion of the organ to a fluid or tissue sample from the patient and measuring heat output from the sample using a calorimeter to thereby detect whether or not there is a rejection reaction against the organ.

The invention also includes a method of determining whether or not a candidate compound has anti-cancer activity comprising adding the compound to a cancer cell sample in vitro and measuring the heat output from the cancer cell using a calorimeter to thereby determine whether or not the candidate compound has anti-cancer activity. The cancer cell may be of any species mentioned herein, but is preferably a human cancer cell.

The invention also provides a method for determining whether an anti-microorganism compound affects the growth of a mammalian or avian animal comprising adding said compound to cells of said mammal and determining whether detecting the rate of growth of the cells by measuring the heat output from the cells using a calorimeter.

The invention also provides a method of determining a suitable administration regimens for a patient comprising taking a sample from a patient on a first administration regimen and detecting the effectiveness of the first administration regimen by measurement of the immune response, microorganism load or numbers of malignant cells using calorimetry, and then optionally if the first administration regimen is found to be of low effectiveness selecting a second administration regimen for the patient. The first and second administration regimen differ in respect to the drug or combination of drugs which are administered. The patient may be infected with HIV.

The invention also provides a method of determining whether an anti-microorganism or anti-infective compound is able to stimulate or suppress the immune system this can be tested by adding the compound to a respiring or growing cell culture optionally at a pre-determined low concentration threshold, such as < 10xMIC, <8xMIC, < 2xMIC, cell culture< 1xMIC to determine whether or not heat output of the microorganism/virus/mammalian cell occurs in response to the addition of the compound, wherein growth of the microorganism is optionally measured by detecting heat output from the microorganism/virus/mammalian cell using a calorimeter, and wherein the compound may be alone or in a combination with 2 or more other anti-microorganism or immuno suppressive or stimulating agents. The threshold may be less than 0.01ug/ml, 0.01 ug/ml to 10ug/ml, such as 0.1 ug/ml to 1ug/ml.

If the said compound is found to cause increased growth of microorganisms at the pre-determined low concentration, then the compound may be administered to an individual suffering from a condition caused by the microorganism or at risk of such a condition, at a high dosage, but optionally not at dosages causing increased respiration of the said compound.

### The compounds investigated/used in the embodiments of the invention

One or more of the compounds may act at the cell wall or membrane, or at an intracellular location in the microorganism, such as at a ribosome, or directly on DNA synthesis. In the case where the microorganism is a virus one or more of the compounds may insert themselves into the virus. The mode of action of one or more of the compounds may not be (previously) known.

The compound may either activate or suppress the immune system either alone or in combination with other agents

The compound may be a biological agent, such as a protein or monoclonal or polyclonal antibody of any origin. The compound may be a nucleic acid. The compound may be a gene fragment. The compound (particularly proteins, peptides and antibodies) may differ in respect of their amino acid sequence, the type or extent of glycosylation (for example differing in the type and number of sugars which are attached) or the type of extent of lipid modification.

Generally the method comprises performing more than one analysis with the compounds, wherein during in each analysis the compounds are added to the sample in different ratios, which would normally be achieved by providing the compounds in different concentrations (i.e. different molarities).

In one embodiment more than one compound may be used. Thus for example in one analysis a first compound will be present at 0.1 to 1% of the molarity of a second compound, or at 1 to 10%, 20 to 30%, 30 to 40 %, 40 to 50 %, 50 to 60%, 60 to 70%, 70 to 80%, 90 to 100%, 100 to 150%, 150 to 250%, 250 to 500%, 500 to 1000%, 1000 to 10,000%. If a first compound is present at a molarity which is 100% of the molarity of the second compound then clearly the compounds are present in a ratio of 1:1 (i.e. an equimolar ratio). Preferably at least 3, such as at least 5, 10, 20, 30 or more analyses are performed with different ratios of the compounds. Typically in the method analyses are performed using at least 2, 3, 5, 10 or more of the specific ratios mentioned above.

### Samples used in the methods of the invention

Any suitable sample from the individual may be used in the methods mentioned herein (for example a sample from a tissue or body fluid which is normally infected by the microorganism or a sample that shows cancerous growth). The sample is typically urine, stools, whole blood, plasma, a body secretion (or a swab thereof) or a tissue sample. The sample may or may not be processed before it is analysed in the method of the invention, but in a preferred embodiment it is not processed before analysis. Such processing typically results in a removal of a component from the sample or may result in dilution of the sample (such as dilution of a swab sample). As discussed below substances such as nutrients may be added to the sample to aid growth of the microorganism. Such additions of substances are not understood herein as being "processing" of the sample.

Typically the sample (at the point of being taken from the individual) has a volume of less than 10 millilitres, such as less than 5, less than 3 or less than 3 millilitres, down to for example 25 ul or less. The sample which is analysed by calorimetry typically has a volume of less than 10 millilitres, such as less than 5, less than 3 or less than 3 millilitres, down to for example 25 ul or less.

### The donor, patient or individual from whom a sample is taken

The donor or patient may be from any species, and is typically a bird or mammal. The individual is preferably human or an animal. The individual may be one which is in a state that renders it susceptible to infection by the microorganism, or susceptible to cancer.

### Microorganisms and cells which are used and/or detected in the methods of the invention

Any suitable microorganism may be used or detected in the methods of the invention. The microorganism is typically a bacterium, yeast, fungus or protozoa or virus. A tumor or malignant cell culture may be used or detected in the method of the invention.

The microorganism may be vancomycin resistant enterococci, pencillin resistant Streptococcus pneumoniae, methicillin resistant Staphylococcus aureus, multi-drug resistant gram-negative bacillus, multi-drug resistant Mycobacterium tuberculosis, multi-drug resistant gram-positive bacteria, Achromobacter, acinetobacter, aerococcus, aeromonas, alicalignes, bacillus, bacteroides, burkholderia, chryseobacterium, citrobacter, clostridium, corynebacterium, enterobacter, enterococcus, escerichia, fusobacterium, haemophilus, hafnia, klebsiella, listeria, micrococcus, moraxella, morganella, pantoea, peptococcus, peptostreptococcus, proteus, providencia, pseudomonas, salmonella, serratia, staphylloccocus,stenotrophomonas, streptococcus multi-drug resistant malaria parasite or HIV, HSV, RSV or viruses which are members of the families Picomaviridae (e.g. polioviruses or rhinoviruses); Caliciviridae; Togaviridae (e.g. rubella virus or dengue virus); Flaviviridae; Coronaviridae; Reoviridae (e.g. rotavirus.); Bimaviridae; Rhabodoviridae (e.g. rabies virus); Orthomyxoviridae (e.g. influenza virus types A, B and C); Filoviridae; Paramyxoviridae (e.g. mumps virus, measles virus, respiratory syncytial virus or parainfluenza virus); Bunyaviridae; Arenaviridae; Retroviradae (e.g. HTLV-I ; or HTLV-II); Papillomavirus, or a tick-bourne encephalitis virus.

The tissue could be from any organ such as liver, kidney , heart, lung etc and from any species

### Conditions under which detection is performed

Typically detection of heat is carried out for at least 2 to 48 hours, such as 3 to 24 or 8 to 12 or 4 to 5 hours (typically after addition of the relevant substance to the calorimeter). Preferably continuous measurement of heat detection is performed, such as at least once every 10 minutes, such as over any of the above specified time periods. For example over a 24 or 48 hour period cell death can be demonstrated (and thus the bacteriocidal effect of antibiotic in vivo), or over a 2 hour period inhibition of growth (and return to baseline) or compatibility of compounds.

The sample may be placed in a temperature which allows growth/heat output of the cell or microorganism to be detected, such as in a temperature of 20 to 45 degrees C, typically in a temperature of 30 to 40 degrees C, such as at a temperature of 35 to 38 degrees C or at 37 degrees C.

The sample is typically placed in conditions allowing growth of the cell or microorganism for 3 to 12 hours, and typically for less than 8 hours, such as less than 6 or less than 4 hours.

As mentioned above in the method heat is detected (for example produced by the respiration, activity or growth of microorganisms may be detected). In one embodiment the heat produced is analysed over time, for example in the form of a graph of heat produced versus time (or power versus time). Such an analysis may allow detection of the quantity of microorganisms present in the original sample or may allow determination of the species of the microorganism. The heat data may be compared to control data to aid in ascertaining microorganism numbers and/or species.

The micro/nano calorimeter which is used in the method of the invention is normally one which is capable of continuously measuring heat effects (enthalpy changes) of a sample. In one embodiment the calorimeter is capable of detecting the heat output of 10² to 10⁵ CFU/ml of microorganisms, such as of any of the species mentioned herein. Typically the calorimeter is capable of detecting a rate of heat change of 1uW or 1nW. The calorimeter may be capable of measuring heat output simultaneously from more than one sample, such as from at least 4, at least 20, at least 50 or at least 100 samples. During analysis of the heat output the sample may be in aerobic or anaerobic conditions.

The invention also provides a kit for carrying out a method according to any of the preceding claims comprising a calorimeter suitable for use in said method. The calorimeter may be specifically adapted so that any of the types of samples mentioned herein can be analysed and/or data concerning heat output from a sample can be provided to a computer or database for analysis of the data.

### Detecting side effects or detrimental effects of anti-microorganism compounds

One aspect of the invention is based on the effect of low concentrations of anti-microorganism compounds on the growth of cells. Such cells can be bacterial cells (such as an any type or species of microorganism mentioned herein), preferably bacterial cells, or eukaryotic cells. It has been found that certain antimicrooorganism compounds, especially compounds that have an effect on RNA synthesis, can cause cells to grow and thus may explain bacterial resistance (increased intracellular protein synthesis providing nutrients for the bacteria in vivo), viral infections (increased synthesis of mRNA and the expression of dormant DNA fragments, e.g. the appearance of shingles after administration of antibiotics) and also the emergence of HIV (a mixture of the two above mechanisms eventually rendering the immune system ineffective). The same effect in eukaryotic cells would give rise to tumour growth. Thus previously observed apparent resistance to anti-microorganism compounds may be due to stimulation of growth of the relevant microorganism at a concentration of anti-microorganism compound which is too low or alternatively due to stimulation of eukaryotic growth when administered in large excess or when no microorganism is present.

The effect of the anti-microorganism compound on cells is consistent with the known growth promoting effects of antibiotics. This also suggests a mechanism for anti-microorganism compounds to cause cancer.

The present work thus provides evidence of actions of anti-microorganism compounds which can cause detrimental effects in the individual being treated. Such actions will contribute for example to some of the side effects observed when using anti-infective compounds.

Accordingly the invention provides a method of determining whether an anti-microorganism compound (e.g. an anti-infective compound) is able to cause increased growth or respiration of the microorganism/virus/cell comprising adding the compound to either a respiring or growing microorganism, optionally at a pre-determined low concentration threshold, such as < 10 X MIC , <8 X MIC, < 2 X MIC, or < 1 X MIC. A single anti-microorganism compound may be added or 2, 3, 4 or more anti-microorganism compounds may be added.

For neomycin (with a known mode of action of cause misreading during RNA synthesis) an increased heat output can be observed at concentrations of < 1ug/ml or in combinations with other antibiotics. For respiring E.coli all concentrations of Neomycin caused an increased heat output.

This can be used to determine whether or not growth of a microorganism/virus or cell occurs in response to the addition of the compound. Preferably growth of the microorganism/virus or cell is measured by detecting heat output from the microorganism/virus or cell using a calorimeter, such as in any of the ways discussed previously. The microorganism is generally any microorganism that causes disease, for example any such microorganism disclosed herein.

If the said compound is found to cause increased growth of microorganisms at the pre-determined low concentration, then appropriate action can be taken in the manner in which it is used, for example it can be administered to an individual suffering from a condition caused by the microorganism or at risk of such a condition, at a high dosage which would ensure killing of the microorganism. It can also be used to monitor infections in patients and thus administer anti-infective agents when necessary and stop treatment when the microorganism/virus or cell is dead (indicated by return to baseline of heat output, confirmed by viable count measurements).

### Anti-microorganism compounds and agents that cause expression of latent DNA

The anti-microorganism compound may be Ampicillin, Chloramphenicol, Erythromycin, Nalidixic acid, Tetracycline, Aminoglycoside (such as streptomycin, kanamycin, gentamicin, tobramycin, amikacin, netilmicin and neomycin), tetracycline, minocycline and doxycycline, Spectinomycin, lincomycin, clindamycin, a macrolide (such as Erythromycin, clarithromycin, azithromycin or spiramycin), Fusidic acid, Rifampin, rifamycin, rifampicin, Quinolone (such as nalidixic acid, ciprofloxacin, oxolinic acid, Ofloxacin, norfloxacin, levofloxacin, lomefloxacin, sparfloxacin), sulfonamide, sulfone, Trimethoprim, methotrexate, pyrimethamine, Para-aminosalicylic acid (PSA), Dapsone, Isoniazid (INH), Spectinomycin, a Penicillin, flagyl metronidazole, Actinomycin, a sulphadrug, vancomycin, adrenamycin, daqucomycin, rifamycin, heparin, afo/3, streptoygidin, or Streptovaricin.

Any of these specific compound may also be used to cause expression of latent DNA, although any suitable agent for causing DNA expression can be used, such as a polymerase enzyme (for example DNA polymerase or RNA polymerase).

### Administration of agents

As mentioned above the invention includes administration of particular agents. Such an agent will generally need to be formulated into an appropriate composition to make it suitable for administration. The agent will typically be administered to an individual (human or animal) in need thereof.

The formulation will depend upon factors such as the nature of the substance and the individual to be treated. Any such substance may be administered in a variety of dosage forms. It may be administered orally, parenterally, subcutaneously, intravenously, intramuscularly, intrasternally, transdermally or by infusion techniques. The substance may also be administered as suppositories.

Typically the substance is formulated for use with a pharmaceutically acceptable carrier or diluent. The pharmaceutical carrier or diluent may be, for example, an isotonic solution.

An effective amount of substance is administered. The dose may be determined according to various parameters, especially according to the substance used; the age, weight and condition of the individual to be treated; the route of administration; and the required regimen. A typical daily dose is from about 0.01 to 500 mg per kg, preferably from about 0.1mg/kg to 10mg/kg of body weight.

The following Examples illustrate the invention:

### Examples

A micro/nano calorimeter is an apparatus that can measure the heat lost or gained during all chemical, physical and biological processes. The heat produced during these processes creates a temperature difference between the sample in the test chamber and its surroundings. The intensity of the process can thus be established through continuous monitoring of the temperature difference. The instrument used in the work is a heat conduction microcalorimeter and is designed to minimise temperature difference and encourage the most rapid heat flow away from the reaction site to a large heat sink (ΔT<10⁻⁴°C).

Micro/nano calorimetry is a non-destructive and non-invasive direct technique that does not require any prior sample treatment (like the lysis-filtration with impedance detection method). The analysis is also not limited to a physical state of a sample i.e. the calorimeter can deal with any sample form: solids, liquids, gases, transparent and non-transparent samples alike can be analysed. The colour of the sample is also not an issue using calorimetry. The calorimeter's indifference to colour and substrate nature makes it an ideal system for analysing the red, complex, suspended, cell composition of blood. Calorimetry provides a favourable advantage to the classical method of blood culturing as it can analyse very small samples of blood.

If an uninfected sample is placed in a calorimeter, one would expect there to be a clinically insignificant number of organisms present per ml (i.e. <10⁴ organisms/ml). It would thus take some time for the organisms in the sample to grow (to >10⁴ organisms/ml) and produce enough heat so that it can be detected in the calorimeter. Conversely, an infected sample will have >10⁴ organisms/ml. When this sample is placed in a calorimeter, it would have enough organisms to generate an immediate detectable level of heat.

The same principle can be used when assessing tissue culture and agents affecting the immune system. The sensitivity, reproducibility and ability to assess mode of action of agents has been demonstrated in an prokaryotic system both assessing growth and metabolism stimulating and repressive agents alone and in combinations

### The Calorimetric Incubation Vessel

The bacterial cultures were grown in a fermenter vessel of approximately 200ml capacity which had a flat ground-glass rim. To the vertical face of the vessel close to its base, equidistantly spaced around the circumference, ports were fitted for an oxygen electrode and for sampling/inoculation. The fermenter was closed with a flat flanged glass lid which had five ports accommodating respectively, gas inlet tube, gas exhaust (via air condenser), inlet and outlet flow-lines to and from the calorimeter. The fermenter and lid was sealed with a p.t.f.e. gasket and clamped with a metal clip. The fermenter was placed on a magnetic stirring unit in a water jacket which allowed circulation of the thermostatically controlled water at 30°C or 37°C. The fermenter which contained a Teflon coated magnetic stirring bar, was sterilised by autoclaving.

### The Calorimetric Incubation

For a typical calorimetric incubation the medium was passed through the calorimetric vessel at the same flow rate and temperature as those used in the experiment to establish a steady baseline deflection. The following operations then took place to a strict timetable. The liquid nitrogen stored ampoule was thawed at 38°C for 3 min and then stirred for 20 sec on a whirlimixer. The bacterial suspension (0.5-1.5ml) volumes was inoculated into the medium (100ml) two minutes after completion of thawing. A Gilson automatic pipette was used for inoculation. The calorimeter inlet flow-line was placed in the incubation medium 30 seconds after inoculation. The flowing incubation medium reached the calorimetric vessel two min after inlet flow-line insertion. At this time the recording of the p-t curves was started. Eight min after inoculation the outflow from the calorimeter was returned to the fermenter (after the "baseline" medium had run to waste but before the inoculated medium ran to waste) thus making a closed system. Aeration was started as soon as the inlet tube was inserted in the fermenter. Microbiological purity of the culture was checked by plating out a sample of the culture at the end of each incubation on nutrient agar (lab M).

The flow-microcalorimeter cannot be sterilised by conventional methods but can be decontaminated by successively pumping through solutions of 0.1 % w/v NaOH and finally sterilised deionised water. (For M. Flavus it was necessary also to use 70% w/v ethanol as well to remove any traces of peptone in the flow lines).

### Presentation of Calorimetric Results

The output of calorimetric experiments is a plot of power (energy evolution per unit of time) as a function of time. The unit of power (p) is the Watt (W) and the unit of time is the hour (h). In some experiments seconds (s) were used as time unit.

### Precision

The accuracy of the flow microcalorimetric assay is high. The parameter most likely to cause any variation in accuracy would be a variation in antibiotic standards. The sensitivity of this assay is also very high. This can be seen from the results of the EM's of the bacteria where no visible damage to the bacteria can be detected within the sensitivity ranges of the assay implying that very small metabolic changes can be detected in the calorimeter. This is important as conventional microbiological assays can not compete with the sensitivity which is obtained at concentrations close to those found *"in vivo".*

Flow - microcalorimetry is a very simple method to use for routine assay of antibiotics, when all the conditions discussed previously are observed. For this assay the standard deviation was ± 2.9% which is a great improvement in reproducibilities from plate assays. (See table ref below).

| Criteria | Calorimetry | Plate Method |
|---|---|---|
| Inter Assay Reproducibiity % | 3 | 5-10 |
| Sensitivity u/ml | 0.1 | 20 |
| Range u/ml | 0.1-100 | 20-100 |
| Time per assay (h) | 1 | 16 |
| Capacity for automation | High | low |
| Convenience and simplicity | High | high |

### EXAMPLE TO DEMONSTRATE THE CAPABILITY OF THE TECHNIGUE TO ASSESS BOTH STIMULATION AND REPRESSION OF GROWTH AND METABOLISM ON RESPIRING OR GROWING CELLS.

### ASSAY OF MIXTURE OF ANTIBIOTICS

Even in the earliest days of the antibiotic era, despite dramatic cures of what had previously been fatal infections, it was apparent that in certain situations the use of a single drug led to unacceptable failure rates.

The exact role of antibiotic combinations in current therapy of clinical infections often remains controversial or unclear, in part because few tightly controlled studies have been carried out in this area.

Compatibility of different antiinfectives or indeed non compatibility involves several mechanisms, including sequential blockage in a given metabolic pathway e.g. trimethoprim and sulfonamides or changes in permeability to the bacterial surface by one drug e.g. penicillin and aminoglycosides or inhibition of inactivating enzymes e.g. penicillin and cloxacillin.

Results have varied when combinations of aminoglycosides and antipseudomonas penicillins have been used against Ps. aeruginosa and when combinations of cephalosporins and aminoglycosides have been used against Klebsiella species. However it is clearly unusual for non compatibility to be suggested by one technique when another method shows compatibility of an antibiotic combination against the same organism.

In the interpretation of the results of all tests of *in vitro* antimicrobial activity, attention must be paid to the overwhelming importance of host factors in the outcome of treatment of any infectious process. *In vitro* tests do not usually take into account the role of phagocytic cells, humoral antimicrobial substances, pharmacokinetic factors and pathopysiological conditions at the site of infections. There are techniques to establish bactericidal effects in blood but these have serious microbiological limitations as regards performance and interpretation of results. The results depend on how the test was done e.g. on the salt concentration and other environmental parameters. However some correlation could be achieved between the test and the clinical outcome.

No clinical studies have been carried out in which an antibiotic combination consistently compatible with another against an infecting organism was compared to a single agent with *in vivo* activity against the infecting pathogen.

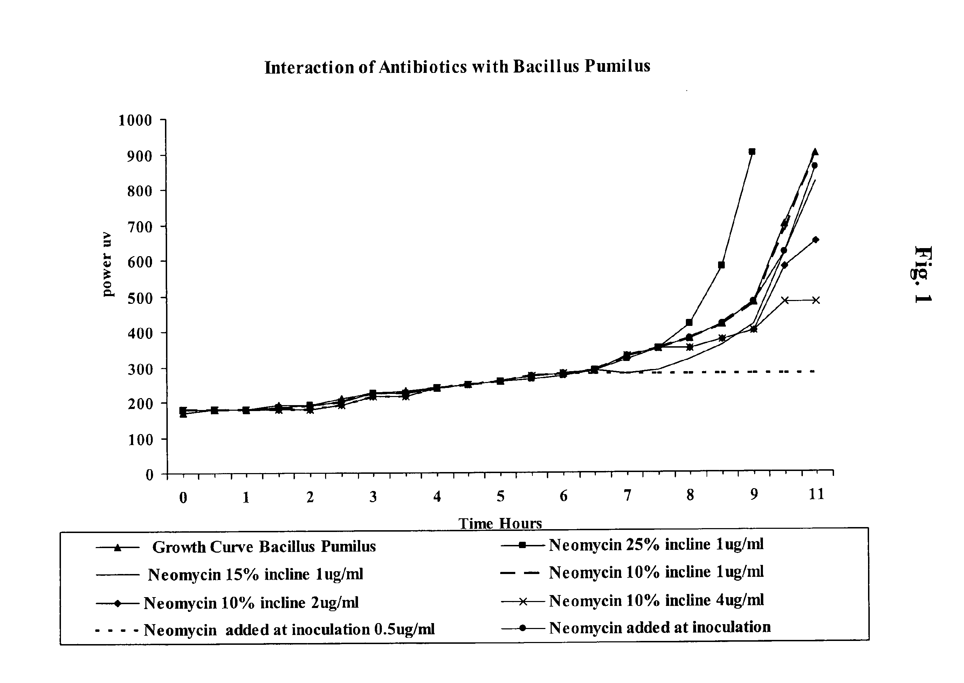

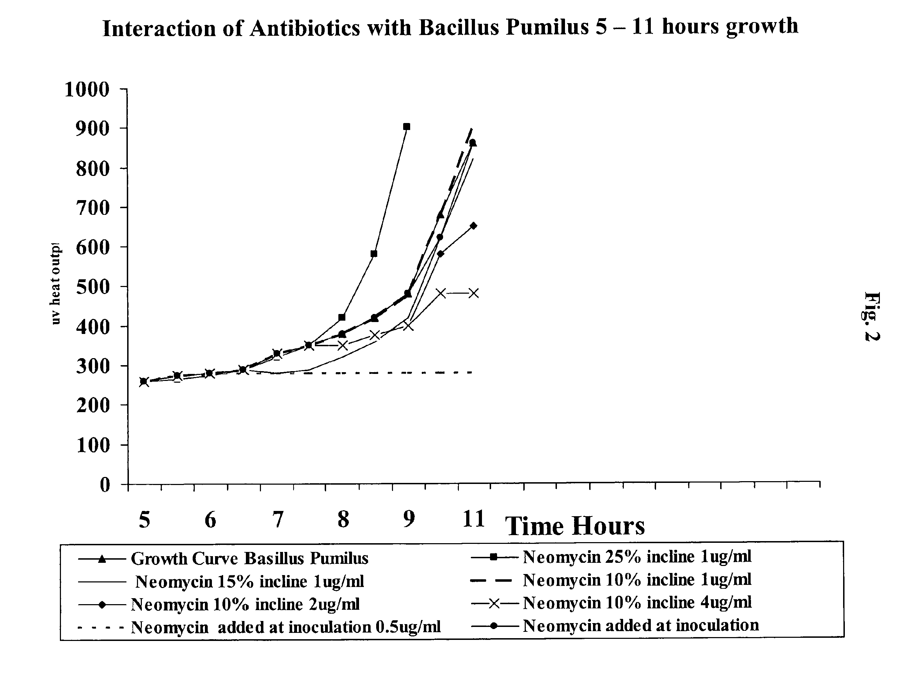

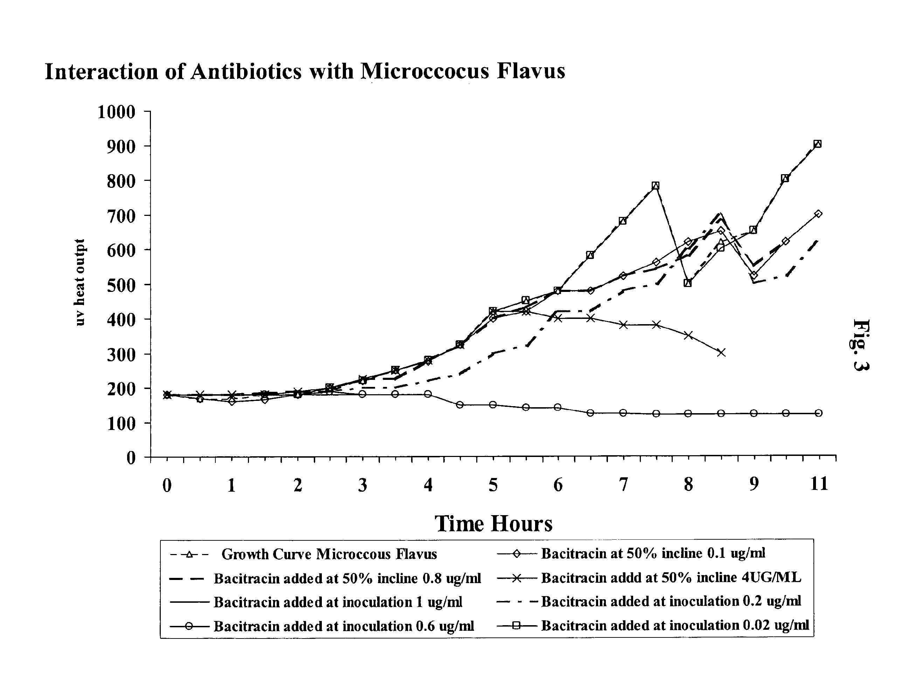

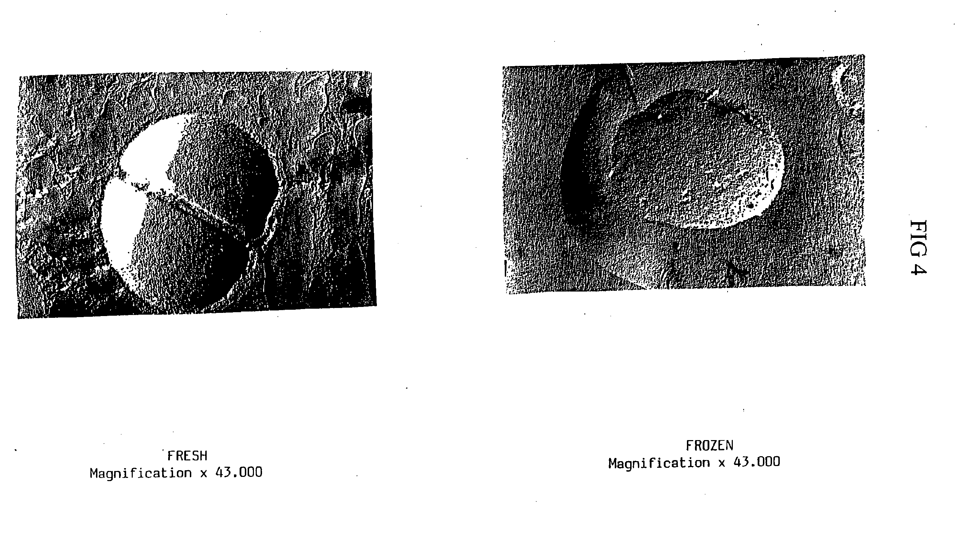

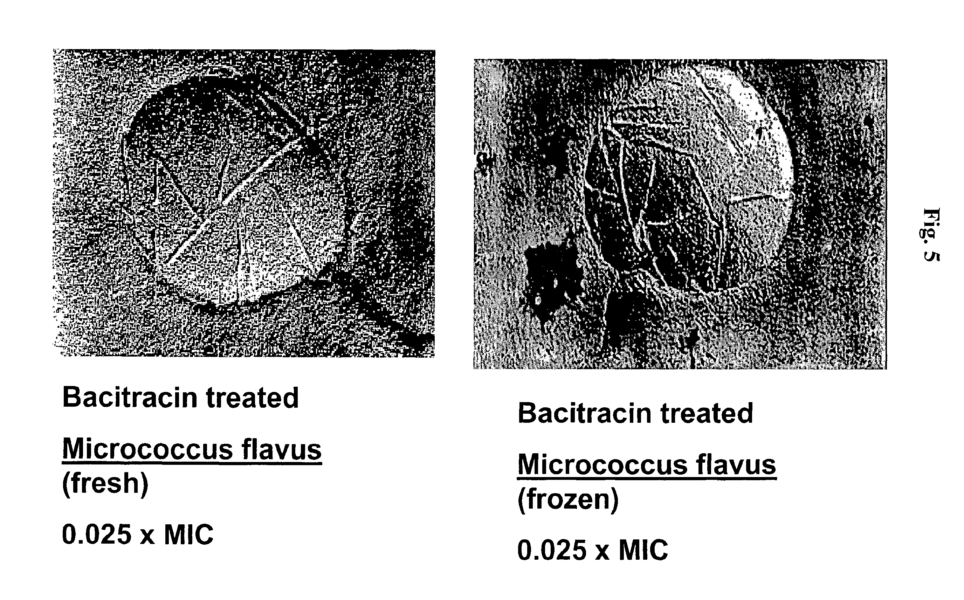

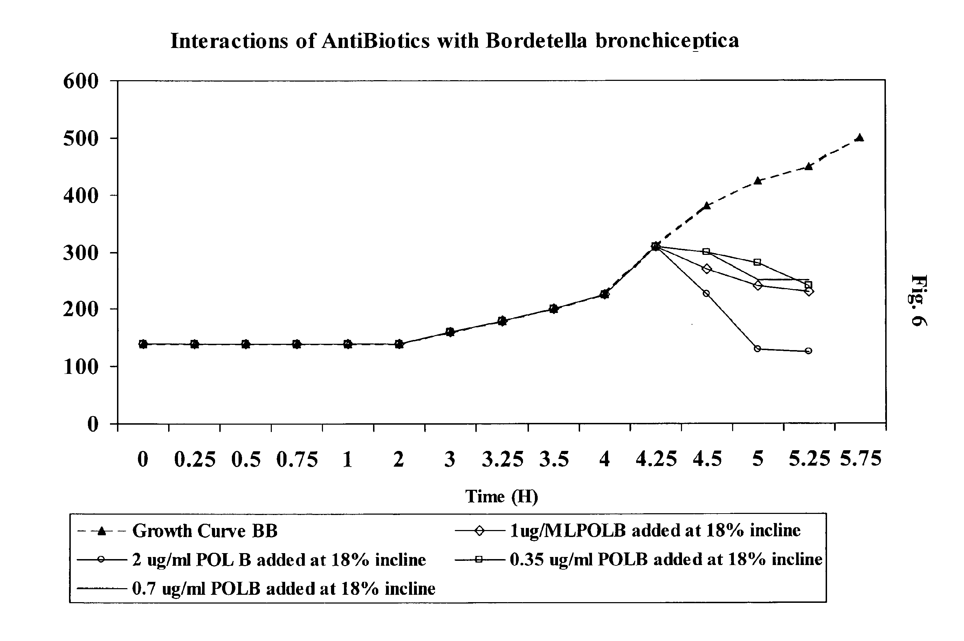

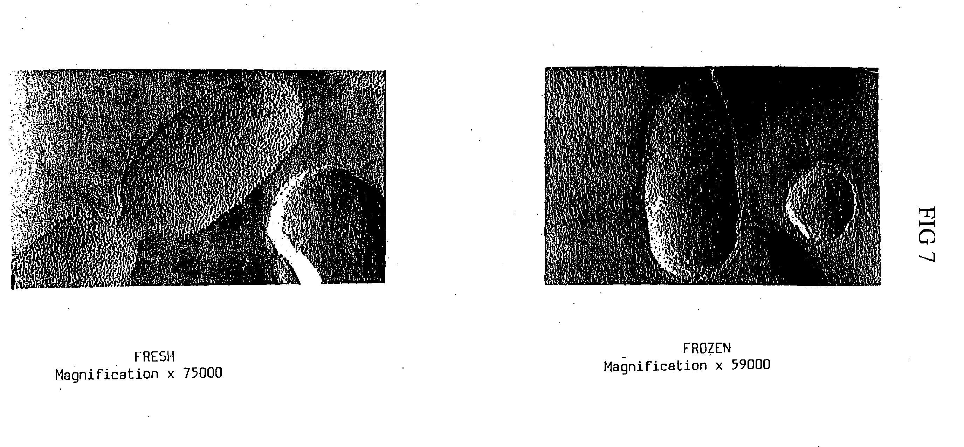

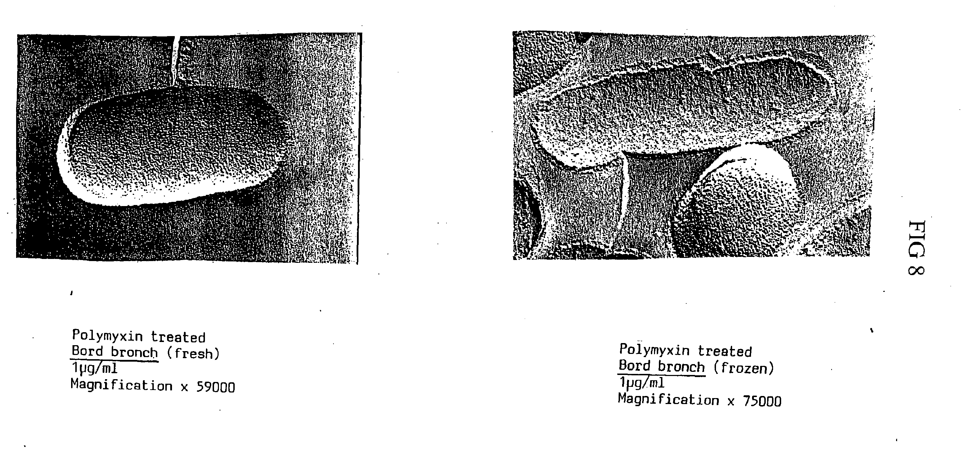

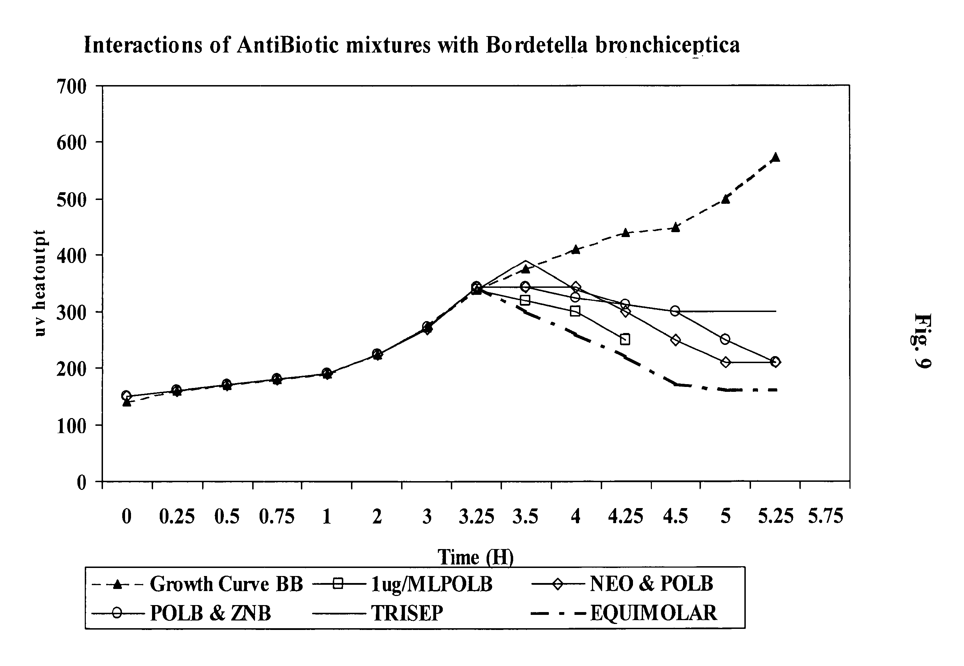

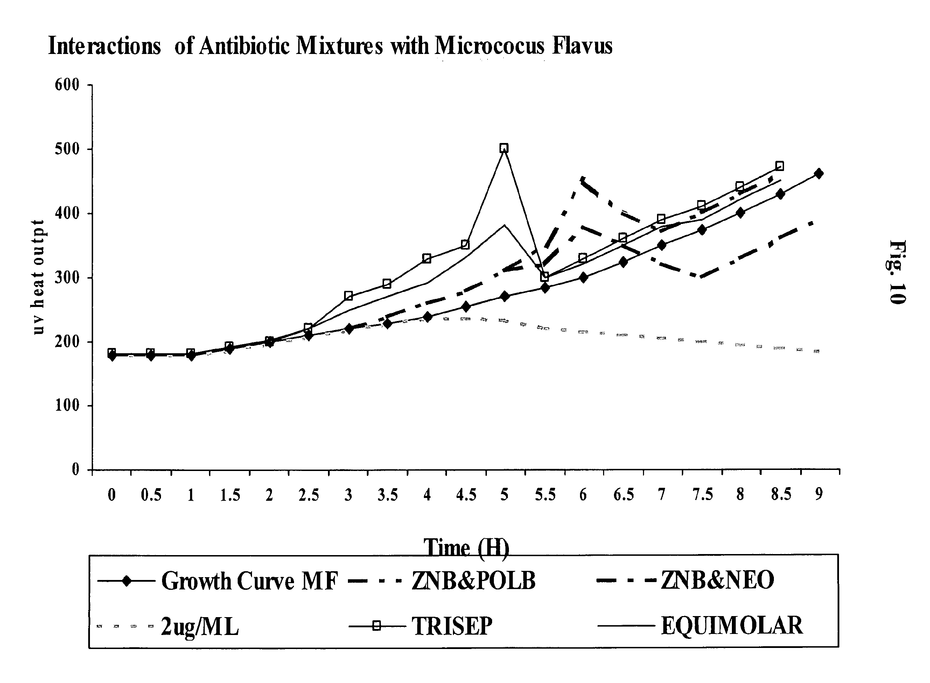

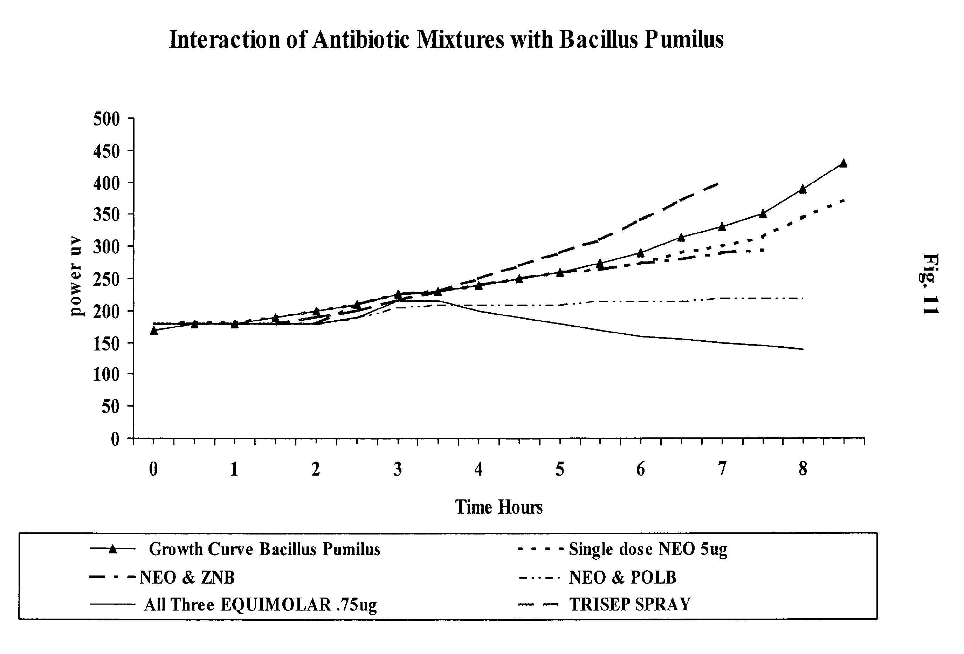

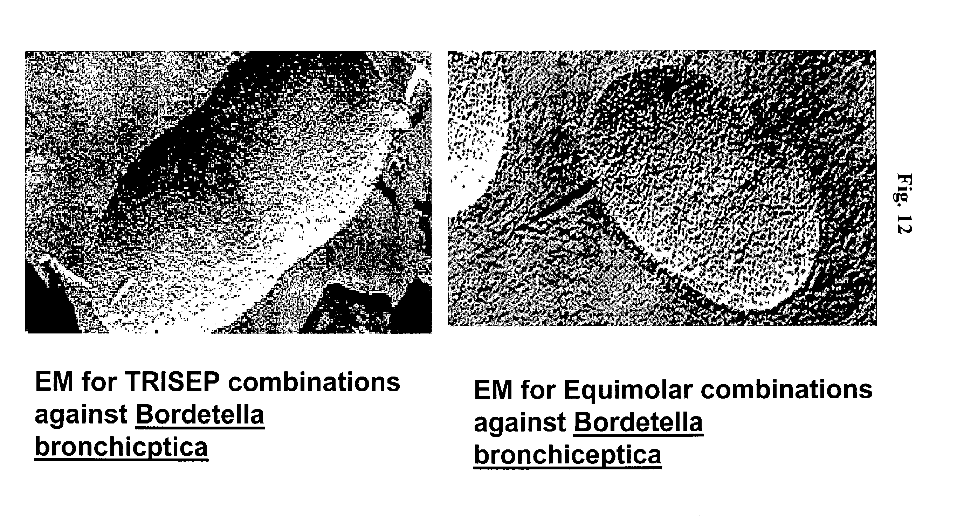

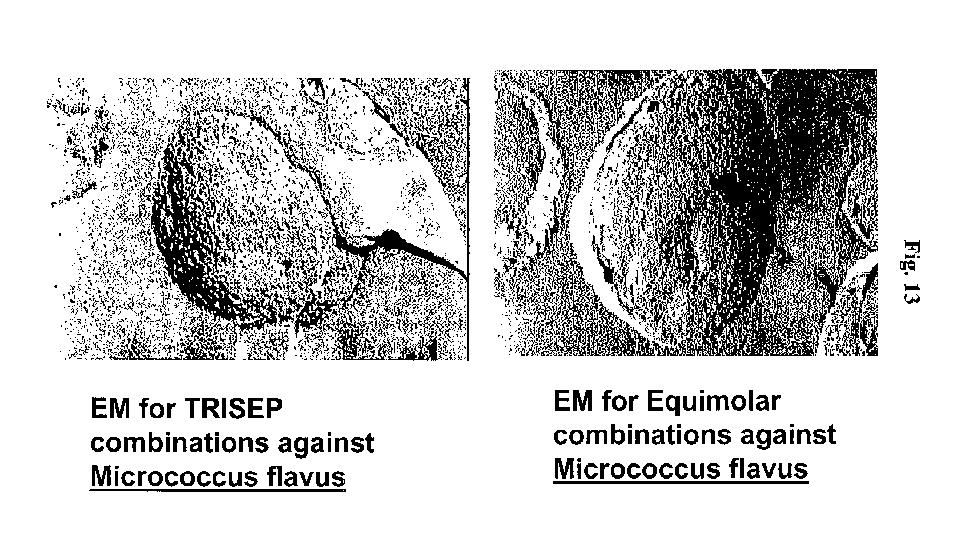

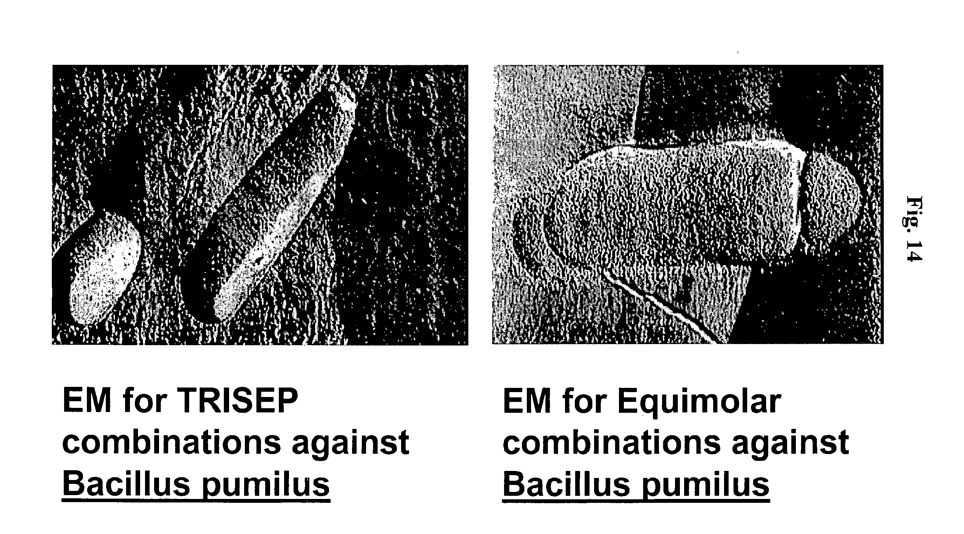

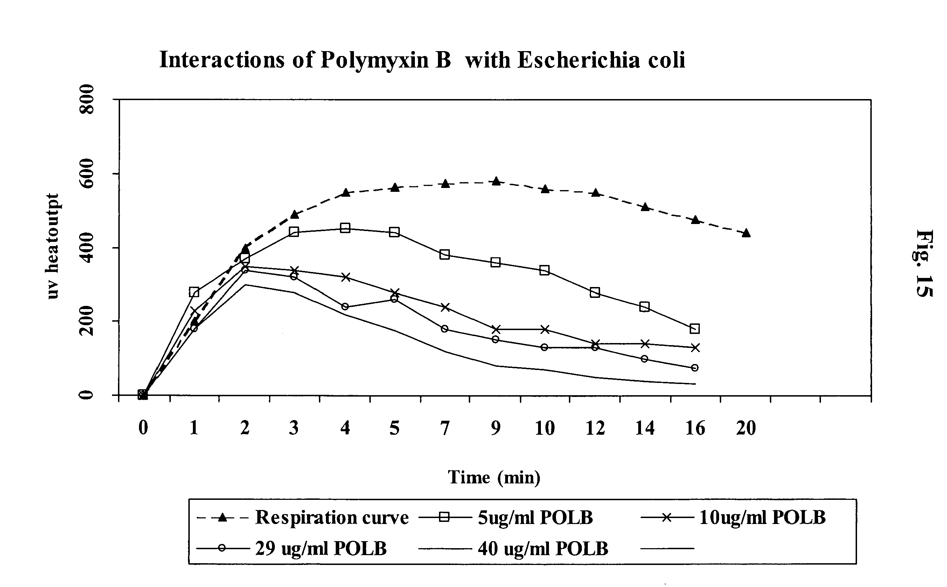

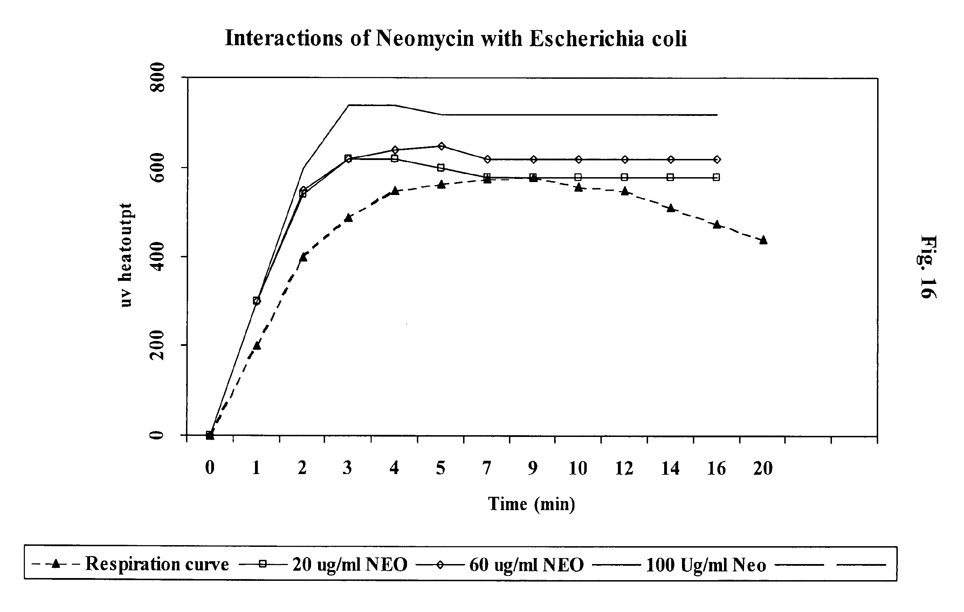

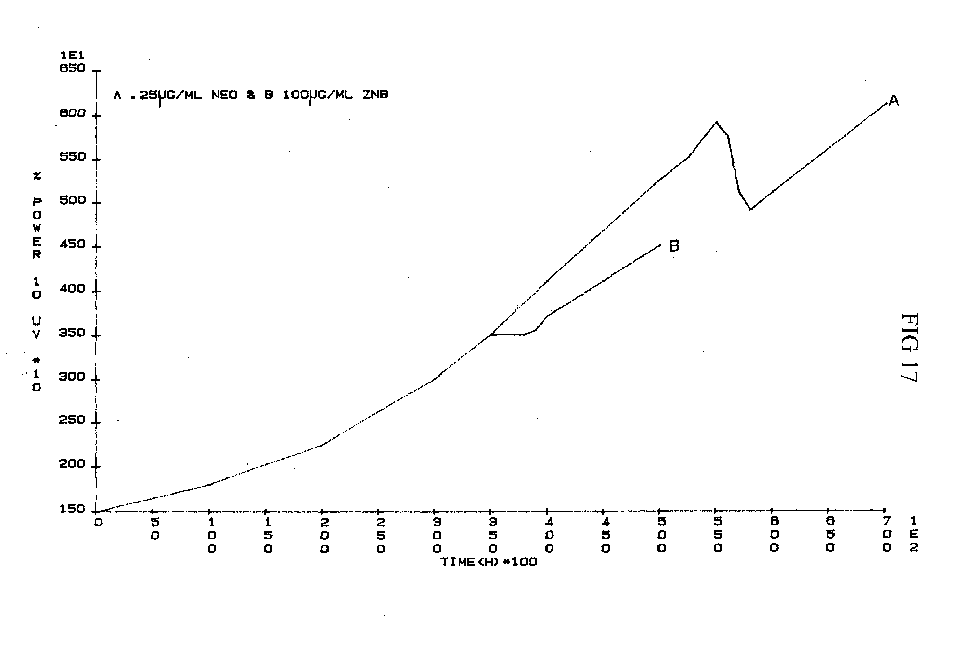

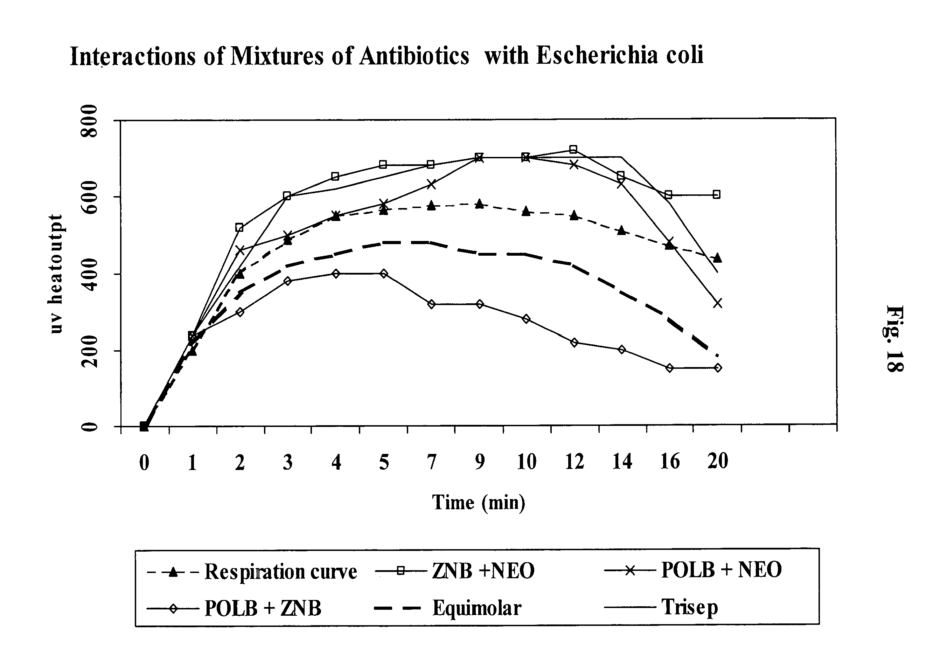

Biological compatibility and non compatibility of antibiotics can be demonstrated by micro calorimetry (see Figures 9 to 18). The antibiotics assayed in combination in this study were POLB, NEO and ZNB. These antibiotics were available as an antibiotic spray "TRISEP" from ICI and contained, on average, NEO 500.00 u, POLB: 150.000 u and ZNB 40000 u per can. Converted to mg the content was NEO 7.5 x 10² mg, POLB 1.7 x 10² mg and ZNB 6.32 x 10² mg. When assaying antibiotics in combination one antibiotic is usually added at some multiple of its MIC, whereas the amount of the second or third antibiotic added is often empirically gauged. A simpler and more regular picture of the interaction of mixtures of antibiotics would be achieved by using equimolar concentrations or concentrations taking into account the mode of action of the antibiotics. In this study antibiotic mixtures have been assayed in the proportions found in the Trisep mixture and in equimolar concentrations. The relative molecular masses (RMM) for the three antibiotics were as follows: ZNB: RMM 1477, POLB: RMM 1300 and NEO RMM 700.

### MIXTURES OF ANTIBIOTICS ASSAYED AGAINST BORDETELLA BRONCHISEPTICA (see Fig. 9)

| **Bacterial Concentration Bordetella Bronchiceptica** | **Antibiotic Concentration** | **Observed effect on p-t curves** | Comments |
|---|---|---|---|
| **As % of p-t curve** | **Ug/ml MIC NEO 0.25 ug/ml ZNB 100ug/ml POL 2ug/ml** | | |
| 50 | 0.25ug/ml Neo | No effect on p-t curve | |
| 50 | 100ug/ml ZNB | Slightly delayed peak | |
| 50 | Pol 1 ug/ml, Neo 4 ug/ml (Trisep conc) | Sharp decline in curve 10 min after addition of antibiotics response polB 0.7 ug/ml | Non compatibility of antibiotics demonstrated |
| 50 | POL 1 ug/ml ZNB 3.57ug/ml | 0.39 ug/ml POLB | Non compatibility of antibiotics demonstrated |
| 50 | All three antibiotics trisep combinations | 0.47 ug/ml Pol B | **2 hours after addition of antibiotic pt-power out put declined 10%, No return to baseline** |
| 50 | All three antibiotics equimolar amounts | 0.39 ug/ml PolB | **2 hours after addition of antibiotic pt-power out put declined 20% No return to baseline** |

| | | | |
|---|---|---|---|
| Fig 9 | | | |

### MIXTURES OF ANTIBIOTICS ASSAYED AGAINST MICROCOCCUS FLAVUS

(see Fig. 10)

| **Bacterial Concentration Micrococcus flavus** | **Antibiotic Concentration** | **Observed effect on p-t curves** | Comments |
|---|---|---|---|
| **As % of p-t curve** | **Ug/ml MIC NEO 4 ug/ml ZNB 0.2ug/ml POL 2ug/ml** | | |
| 0? | 4 ug/ml Neo | No effect on p-t curve | |
| 0? | 2ug/ml POLB | Slightly delayed peak | |
| 50 | 0.2 ug/ml ZNB 056 ug/ml POLB (trisep) | Delay in peak ZNB conc 0.11 ug/ml | Non compatibility of antibiotics demonstrated |
| 50 | NEO 0.226 ug/ml ZNB 0.2ug/ml (trisep) | 0.1 ug/ml ZNB | Non compatibility of antibiotics demonstrated |
| 50 | All three antibiotics trisep combinations | 1.3 times standard cuve | **Growth promoting effect** |
| 50 | All three antibiotics equimolar amounts | 0.1 ug/ml ZNB | **Small delay in peak compared to standard curve** |

| | | | |
|---|---|---|---|
| Fig 10 | | | |

### MIXTURES OF ANTIBIOTICS ASSAYED AGAINST BACILLUS PUMILUS

(see Fig. 11)

| **Bacterial Concentration Bacillus Pumilus** | **Antibiotic Concentration** | **Observed effect on p-t curves** | Comments |
|---|---|---|---|
| **As % of p-t curve** | **Ug/ml MIC NEO 1 ug/ml ZNB 20 ug/ml POL 20 ug/ml** | | |
| 0? | ZNB 20 ug/ml | No effect on p-t curve | |
| 0? | 20ug/ml POLB | Within 9 hours growth was inhibited completely | |
| 0 | 0.5 µg/ml) NEO + 0.125 µg/ml (0.006 MIC) POLB (TRISEP) | Compatibility Effect 1ug/ml neomycin | compatibility of antibiotics demonstrated |
| 0 | And 0.25 MIC (0.5 µg/ml) NEO + 0.44 µg/ml 0.022 MIC ZNB (TRISEP). (trisep) | Compatibility Effect 1 ug/ml neomycin | compatibility of antibiotics demonstrated |
| 0 | All three antibiotics trisep combinations | 1.0 times standard cuve | **No effect** |
| 0 | All three antibiotics equimolar amounts neomycin 1 ug/ml, POLB,ZNB | 1.7ug/ml Neomycin | **Compatibility demonstrated** |

| | | | |
|---|---|---|---|
| Fig 11 | | | |

### DISCUSSION

Compatibility was clearly demonstrated for all antibiotic combinations against B. pumilus except the Trisep mixture. These studies were all carried out at pH 8.00 as this was the most effective pH for neomycin. It seems likely that when using the Trisep mixture POLB and ZNB interfered with each other whereas an equimolar concentration no such effect was seen. The results suggest a critical range of concentration within which compatibility is observed.

When assaying the three antibiotics against Bord. Bronch and M. flavus no such effect could be observed. In fact an indication of non compatibility could be observed for all these mixtures, and even a growth promoting effect at lower concentrations of antibiotics. It seems likely that the compatible action against Bacillus pumilus was due to POLB and ZNB facilitating the transport of neomycin into the cytoplasm by disrupting the bacterial membrane. All the above results have been verified by electron microscopy (see Figs. 12 to 14). It has been shown by EM that ZNB had some effect on E. coli as protrusions have been observed on the bacterial membrane after treatment with ZNB.

A possible assay of POLB and ZNB in the combinations used here could probably be achieved using Bord. bronch and M. flavus respectively as test organism. The assays would not be as sensitive as the assays for the individual antibiotics but a linear relationship between dose and response may be achievable. For the neomycin assay, using B. pumilus as test organism, a great variation in concentration of the antibiotics would have to be carried out to see at which concentrations, if any, an assay of all three antibiotics would be possible i.e. that an assay could be developed for neomycin with a constant response for ZNB and POLB.

### RESPIRATION AND GROWTH OF E.COLI

E.coli was suspended in 10% DMSO prior to freezing and the viable counts were for batch 1: pre-freeze: 4.0 ± 0.6_ x 10¹⁰ cfu/ml and post -freeze 2.0 ± 0.44 x 10¹⁰ cfu/ml and for batch 2: pre-freeze: 3.45 ± 0.58 x 10¹⁰ cfu/ml and post-freeze 2.93 ± 0.43 x 10¹⁰ cfu/ml. The freezing rate for both batches was 20°C/min the survival rate was 50% for batch 1 and 85% for batch 2. These differences may have been due to batch 1 being kept for 24h at 4°C before being frozen.

The respiration experiments were carried out in glucose buffer at different pH. The respiration curves obtained from batch 1 (inoculum 2 x 10¹⁰ cfu/ml) and batch 2 Inoculum 3.2 x 10¹⁰ cfu/ml) were essentially similar and a mean was used as a reference). The respiration experiments were carried out at pH 7 for polymyxin and Znbacitracin and at pH 8 for neomycin. No differences in p-t control curves could be observed at pH 7 or pH 8.

The p-t curves rose and reached a plateau as has been observed for respiration of yeasts and some 20 min later fell to a lower plateau value. Prior incubation of the cells in the buffered glucose for periods up to 20 min before presentation to the calorimeter could reduce or entirely remove this effect.

### INTERACTION OF POLYMYXIN B, NEOMYCIN AND ZNBACITRACIN WITH RESPIRING

E. COLI

| **Bacterial Concentration E. coli** | **Antibiotic Concentration** | **Observed effect on p-t curves** | Comments |
|---|---|---|---|
| **As % of p-t curve** | **Ug/ml MIC&MBC NEO 5900 ug/ml ZNB > 1000 ug/ml POL 6.3ug/ml** | | |
| Respiration | **POLB 6.3 ug/ml** | Reduced height of p-t curve initially and returned to baseline within 1 hour | **Bacteriocidal action** demonstrated |
| Respiration | NEO 500 ug/ml | Increase in heat output Linear dose-response between 10-100ug/ml | **Consistent with increased rate of RNA synthesis** |
| Respiration | ZNB 300 ug/ml - 1000 ug/ml | No response | Resistance demonstrated |
| Respiration | : POLB: 20µg/ml NEO: 80.8µg/ml and ZNB 71.4µg/ml (TRISEP combination). | POLB - NEO longer plateau drop after 20 min to baseline POLB ZNB NEO ZNB NEO response curve | POLB/NEO interaction: delayed response compared to polymyxin alone but drop to baseline no increase in power NEO/ZNB no interference of ZNB with NEO action higher output but not as high as with NEO alone |
| Respiration | All three antibiotics trisep combinations | Polymyxin curve with delayed return to baseline 20 min | **Effect not as good as polymxin alone or Neomycin alone** |
| Respiration | All three antibiotics equimolar amounts | Polymyxin curve with delayed return to baseline 9 min | **Effect not as good as polymxin alone or Neomycin alone** |

Fig 15,16 and 18

### Interaction of Polymyxin B with E.coli

The MIC and MBC for E.coli was found to be 6.3µg/ml. Polymyxin B reduced the initial peak height and thereafter the p-t curve returned to baseline (Fig. 15). This effect was in accordance with the mode of action of polymyxin as the bacteriocidal action was very rapid. This was also observed when using Bord. Bronch as test organism.

### Interaction of Neomycin with E.coli

The effect of neomycin on E.coli is given in Fig. 16. In contrast to polymyxin, neomycin caused an increase in power which confirms that Neomycin fundamentally stimulates RNA synthesis and only inhibits it when present in very large amounts when added to respiring E.coli. (MIC: 500µg/ml). A linear relationship between dose and the reciprocal of increase in response from the control curve was found between 20-100µg/ml neomycin.

### Interaction of Znbacitracin with E.coli

No response could be observed on addition of Znbacitracin to respiring E.coli. The highest concentration added was 300µg/ml (MIC> 1000µg/ml). ZNB has been reported to be inactive against E.coli. However electron micrographs have shown that ZNB had an observable effect on E.coli i.e. did actually cause protrusions to occur on the bacterial membrane. This response was not lethal for the bacterium but small changes in heat could possibly have been expected to occur. This has in fact been observed when ZNB was added to growing cultures of Bord. bronch. a small plateau was reached before growth reoccurred (see Fig. 17).

### Interaction of Mixtures of Antibiotics

Fig. 18 shows the p-t curves for the effect of mixtures of antibiotics on E.coli. The concentrations were: POLB: 20µg/ml NEO: 80.8µg/ml and ZNB 71.4µg/ml (TRISEP combination). Fig. 18 shows the p-t curve for ZNB + NEO; when compared with the p-t curves for neomycin alone it can be seen that this p-t curve was essentially similar to that for neomycin alone (20µg/ml NEO). Fig. 18 shows the effect of POLB + NEO on E.coli. When comparing the effect of the mixture to that of POLB alone (Fig. 15) it can be seen that the effect of the two antibiotics was not comparable to the polymyxin interactions as the kinetics of the interactions seemed to have changed resulting in a plateau value for much longer periods than found for the polymyxin alone followed by a decline to a baseline value after 20 min. When compared to the neomycin interactions (Fig. 16) the p-t curve for NEO and POLB was very different.

Fig. 18 shows the effect of polymyxin and Znbacitracin in combination and when compared with that for polymyxin alone (Fig. 9) the p-t curve is seen to be similar in shape to that for polymyxin but shows a higher output. This response was equivalent to a response of 15µg/ml polymyxin and suggests non compatibility.

Fig. 18 shows the effects of all three antibiotics added to respiring E.coli (A) as the TRISEP mixture and (B) as the equimolar mixture. The TRISEP mixture shows a response similar to that of POLB + NEO apart from showing a decline to baseline which was at a slower rate whereas the equimolar mixture at (20µg/ml POLB) showed a polymyxin type curve with a plateau value very similar to that of the control curve, but then, after 9 min, a steady decline to baseline.

The heat effects observed with polymyxin B were those that would be expected of membrane active antibiotics; i.e. severe reduction in power output followed by a return to baseline signifying death or lysis of cells after a breakdown in membrane potential and leakage of cell constituents from the cytoplasm of the cell. The polymyxin type interactions showed kinetics which were essentially simply and which have been observed with antifungal antibiotics. Neomycin, in contrast, caused an increase in power output also observed in growth studies with Basillus Pumilus which would indicate an immediate effect on RNA synthesis and a very good reason not to use this antibiotic

That ZNB had no effect on power output was not surprising since the antibiotic has been reported to be active only against Gram positive organisms. The assays for POLB were not particularly sensitive (3µg/ml) but had the advantage of being rapid. They do compare favourably with the assay using Bord. Bronch. as test organism for this reason. Which assay to use would depend entirely on the sensitivity required. The reproducibilities for the E.coli assay were also smaller than those for the Bord. bronch. assay. The neomycin assay was much more sensitive when using B.pumilus as test organism. However the assay with E.coli was possible at concentrations much lower than the measured MIC (500 µg/ml). It can be concluded that these two assays would be suitable for screening antibiotics where sensitivity is not essential, since they are very rapid (1h).

It can be concluded from Fig. 18 that all the antibiotics assayed together show (two by two) possible antagonistic effects. The effect observed with neomycin and polymyxin was an initial increase in peak deflection followed by a gradual decrease to baseline. This suggested that the initial effect on E.coli was due to neomycin followed by the effect of polymyxinFor POLB + NEO assayed against E.coli it can be observed that the rate of decline of power was identical to that noted for the antibiotic on its own suggesting that these interactions followed the same order of kinetics at this point. That polymyxin and bacitracin assayed together showed non compatibility was not surprising since a possible explanation could be that competition for binding sites on the bacterial membrane would occur. This effect was also in agreement with the effect observed for this mixture when assayed against Bord. bronch. and M. flavus. This possible competitive effect could result in the inhibition of binding of the most active agent. When all three antibiotics were added together either as the TRISEP combination or as the equimolar combination, p-t curves resulted that were different from those observed either for the antibiotics added singly or in binary combinations.

These results form an excellent basis for rapid compatibility testing of anti-infective agents both during development and also as part of clinical practise in hospitals, doctors offices, dental clinics, veterinary clinics and in development countries

## Claims

1. Method of determining whether or not an organ for transplantation (from a human donor) or xenotransplantation (from an animal donor) is infected with a microoorganism (which is optionally a virus) comprising
- measuring heat output from the organ to detect activity of said microorganism, or
- forcing expression of latent DNA by using agents that stimulate m-RNA synthesis,
to thereby determine the presence of a microorganism in the organ.

2. Method of determining the suitability of a donor organ for transplantation to a patient comprising adding in vitro a portion of the organ to a fluid or tissue sample from the patient and measuring heat output from the sample using a calorimeter to thereby detect whether or not there is a rejection reaction against the organ.

3. Method of determining whether or not a candidate compound has anti-cancer activity comprising adding the compound to a cancer cell sample in vitro and measuring the heat output from the cancer cell using a calorimeter to thereby determine whether or not the candidate compound has anti-cancer activity.

4. Method for determining whether an anti-microorganism compound affects the growth of a mammalian or avian animal comprising adding said compound to cells of said mammal and determining whether detecting the rate of growth of the cells by measuring the heat output from the cells using a calorimeter.

5. Method of determining a suitable administration regimen for a patient comprising taking a sample from a patient on a first administration regimen and detecting the effectiveness of the first administration regimen by measurement of the immune response, microorganism load or numbers of malignant cells in a sample from a patient on the first administration regimen, wherein measurement is performed by using calorimetry, and then optionally if the first administration regimen is found to be of low effectiveness selecting a second administration regimen for the patient

6. Method according to claim 5 wherein the first and second administration regimen differ in respect to the drug or combination of drugs which are administered.

7. Method according to claim 5 or 6 wherein the patient is diagnosed to be HIV positive and antiretroviral agents are used or being developed.

8. Method of determining whether an anti-microorganism or anti-infective compound is able to cause growth of the microorganism/virus/mammalian cell comprising adding the compound to the microorganism, optionally at a pre-determined low concentration threshold, such as < 10xMIC, <8xMIC, < 2xMIC, < 1xMIC to determine whether or not growth or heat output of the microorganism/virus/mammalian cell occurs in response to the addition of the compound, wherein growth of the microorganism is optionally measured by detecting heat output from the microorganism/virus/mammalian cell using a calorimeter, and wherein the compound may be alone or in a combination with 2 or more other anti-microorganism or anti-infective agents.

9. Method according to claim 8 wherein if the said compound is found to cause increased growth of microorganisms at the pre-determined low concentration, then administering the compound to an individual suffering from a condition caused by the microorganism or at risk of such a condition, at a high dosage, but optionally not at dosages causing increased respiration of the said compound.

10. Method according to any one of the preceding claims wherein the donor or patient is a human, animal, mammal, primate, bird, domestic pet, agricultural animal, wild animal, sporting animal or fish or where the sample or cell is from an individual of any such organism.

11. Method according to any one of the preceding claims wherein the donor or patient is of any of the following species, or where the sample or cell is from an individual of any of the following species: horse, pig, cow, dog, cat, sheep, goat, turkey, chicken, camel, llama, deer, duck, fish, monkey, mouse, rat, guinea pig or hamster.

12. Method according to any one of the preceding claims wherein heat output is measured using calorimeter able to detect changes in heat flow of 25 nanocalories/sec and/or heat effects of 10 microcalories.

13. Kit for carrying out a method according to any of the preceding claims comprising a calorimeter suitable for use in said method.
